# EUROPÄISCHE PATENTANMELDUNG

(11) **EP 4 082 474 A2**
(43) Veröffentlichungstag der Anmeldung: **02.11.2022**
(21) Anmeldenummer: 22171020.5
(22) Anmeldetag: 29.04.2022
(51) Int. Cl.: A61B 90/00, A61B 34/20

(54) **INTRAOPERATIVE ERWEITERTE REALITÄT**

(30) Priorität: 29.04.2021 DE 102021111146
(71) Anmelder: mediCAD Hectec GmbH, DE-84032 Altdorf (DE)
(72) Erfinder: ERDMANN, Eric, 84186 Vilsheim (DE)
(74) Vertreter: LS-MP von Puttkamer Berngruber Loth Spuhler

(57) **Zusammenfassung**

Verfahren, bei dem zur Durchführung einer Operation an einem menschlichen oder einem tierischen Körper, einem Visualisierungsmittel zur Kombinierung von natürlichen Wahrnehmungen wenigstens eines Bereiches des Körpers eines Benutzers mit wenigstens einer künstlich erzeugten Darstellung, zur Erzeugung der künstlichen Darstellung Daten bereitgestellt werden, wobei die künstliche Darstellung eine Referenzdarstellung zu dem Bereich des Körpers ist und die künstliche Darstellung zur Durchführung der Operation mit Hilfe des Visualisierungsmittels optisch auf die natürliche Wahrnehmung des Bereichs des Körpers projiziert wird.

## Beschreibung

Die Erfindung betrifft ein Verfahren zur Durchführung einer Operation an einem menschlichen oder einem tierischen Körper nach den Merkmalen des Anspruchs 1.

Die Erfindung betrifft weiter eine Verwendung des zugehörigen Visualisierungsmittels nach den Merkmalen des Anspruchs 14 sowie ein System zur Ausführung des Verfahrens nach den Merkmalen des Anspruchs 15.

Aus der DE 10 2016 218 364 A1 ist ein Verfahren zur Durchführung eines Vorgangs an einem Patienten mittels eines medizinischen Systems bekannt. Das Verfahren umfasst die Schritte: Bestimmen von Sollwerten für jeden Teilschritt des Vorgangs, Erfassen von Istwerten für jeden Teilschritt des Vorgangs, Vergleichen der Sollwerte mit den Istwerten für jeden Teilschritt des Vorgangs, um eine Abweichung zwischen den Sollwerten und den Istwerten zu bestimmen.

Es wäre wünschenswert, einem Operateur für die Durchführung einer Operation am tierischen und/oder menschlichen Körper ein Verfahren bereitstellen zu können, mit dem Daten und/oder Informationen, die während einer Planungsphase für die Operation erzeugt werden, dem Operateur während der Operation wenigstens in zweidimensionaler Form zur Verfügung gestellt werden können.

Die Aufgabe wird gelöst durch ein Verfahren, bei dem zur Durchführung einer Operation an einem menschlichen oder einem tierischen Körper einem Visualisierungsmittel zur Erzeugung der künstlichen Darstellung Daten bereitgestellt werden. Das Verfahren kombiniert natürliche Wahrnehmungen wenigstens eines Bereiches des Körpers eines Benutzers mit wenigstens einer künstlich erzeugten Darstellung.

Die künstliche Darstellung ist als Referenzdarstellung zu dem jeweiligen Bereich des Körpers ausgebildet.

Die künstliche Darstellung wird zur Durchführung der Operation mit Hilfe des Visualisierungsmittels optisch auf die natürliche Wahrnehmung des Bereichs des Körpers projiziert.

### Referenzdarstellung

Die Erfindung versteht unter dem Begriff Referenzdarstellung eine künstliche Darstellung, mit der die natürliche Wahrnehmung des jeweiligen Bereichs des menschlichen oder tierischen Körpers verglichen werden kann.

Die Referenzdarstellung zeigt vorzugsweise eine künstliche Darstellung wenigstens eines Ausschnitts des Bereiches des Körpers, der der Operation unterzogen werden soll.

Die Referenzdarstellung kann einen Wirbel und/oder einen Knochen eines menschlichen Skeletts zeigen. Die Referenzdarstellung kann einen einzelnen Bereich des Körpers (z. B. Wirbel) in Kombination mit wenigstens einem anderen Bereich des Körpers (z. B. Hüftgelenk) zeigen. Es versteht sich von selbst, dass jeder Bereich des menschlichen Körpers alleine oder zusammen mit wenigstens einem anderen Bereich des menschlichen Körpers in Form einer künstlichen Darstellung einer Referenzdarstellung gezeigt werden kann.

Die Referenzdarstellung wird dem Operateur mithilfe des Visualisierungsmittels optisch auf die natürliche Wahrnehmung des jeweiligen Bereichs des Körpers projiziert.

Die künstliche Darstellung kann als Referenzdarstellung mithilfe des Visualisierungsmittels über oder unter oder an wenigstens einer Seite relativ zur natürlichen Wahrnehmung des jeweiligen Bereichs des Körpers projiziert werden.

Bei einer Abweichung einer optischen Projektion der künstlichen Darstellung von der natürlichen Wahrnehmung des Bereichs des Körpers durch den Benutzer löst das Visualisierungsmittel eine optische oder akustische Warnmeldung aus.

Die Abweichung der optischen Projektion kann sich auf die Länge und/oder die Breite und/oder die Dicke des Gegenstands beziehen, dessen natürliche Wahrnehmung mit seiner künstlichen Darstellung verglichen wird.

Die Referenzdarstellung kann relativ zur entsprechenden natürlichen Wahrnehmung des Bereichs des Körpers in einem Verhältnis von 1:1 dargestellt werden. Die künstliche Darstellung kann als Referenzdarstellung relativ zu der natürlichen Wahrnehmung des jeweiligen Bereichs des Körpers verkleinert oder vergrößert dargestellt werden.

Die Referenzdarstellung kann zur Vorbereitung und/oder zur Durchführung und/oder zur Kontrolle der Operation für den jeweiligen Operateur beispielsweise einen Bohrkanal zeigen oder umfassen. Der Bohrkanal gibt die Länge und/oder den jeweiligen Durchmesser der Bohrung an. Er zeigt den Eintrittswinkel der Bohrung in den jeweiligen Bereich des Körpers an, der einer Operation unterzogen werden soll. Die Referenzdarstellung des Bohrkanals dient dem Operateur als Hilfestellung. Mithilfe der Referenzdarstellung kann der Operateur insbesondere sicherstellen, dass die Schraube, die er zur Durchführung der Operation verwenden möchte, die richtige Länge oder die richtige Stärke oder den richtigen Eintrittswinkel in den Knochen aufweist. Es versteht sich von selbst, dass die Referenzdarstellung jeden Bereich des menschlichen oder tierischen Körpers darstellen kann. Die Referenzdarstellung kann zumindest einen Bereich vorzugsweise eines Implantats zeigen.

Die Daten zur Erzeugung der künstlichen Darstellung werden von einer Bildassistenzeinrichtung bereitgestellt.

Die künstlich erzeugte Darstellung kann eine Skala sein. Es versteht sich von selbst, dass die künstlich erzeugte Darstellung auch ein anderes Maßsystem sein kann.

Die künstlich erzeugte Darstellung kann eine Schnittlinie sein, die dem Operateur als Referenzdarstellung für die Führung eines Schnittes in einem Bereich des Körpers dient. Sie kann auch eine Bohrlinie sein. Die Bohrlinie gibt an, in welche Eindringtiefe ein Loch z.B. in einen Knochen gebohrt werden muss, um vorzugsweise ein Implantat in den Bereich eines Körpers einsetzen zu können. Die künstlich erzeugte Darstellung gibt den Eintrittswinkel vor, in dem vorzugsweise eine Schraube und/oder ein Implantat in einem Bereich des Körpers angeordnet werden soll.

Die künstlich erzeugte Darstellung zeigt die wenigstens zweidimensionale Abbildung wenigstens eines Implantats.

Mit Hilfe der künstlich erzeugten Darstellung kann vorzugsweise, aber nicht ausschließlich, wenigstens ein Implantat aus einer Anzahl von mehreren Implantaten ausgewählt werden. Die Implantate können sich in Art, Umfang und/oder Material unterscheiden.

Die Referenzdarstellung gibt den Zustand des Bereichs des Körpers wieder, dem der jeweilige Bereich in einer Pre-operativen Phase entspricht.

Die künstliche Darstellung wird vorzugsweise mit Hilfe wenigstens eines Referenzmarkers, insbesondere deckungsgleich, auf die natürliche Wahrnehmung des Bereichs des Körpers projiziert.

### Operation und Maßnahmen sowie Arbeitsschritte zur Durchführung der Operation

Gegenstand einer präoperativen Planung können operative Eingriffe sein. Beispielhaft, aber nicht ausschließlich, umfassen operative Eingriffe Knochenresektionen, das Einsetzen von Implantaten sowie die Auswahl von Implantaten nach bestimmten Parametern. Die Parameter können die Größe und der Typ des Implantats sein. Die Parameter können verschiedene geometrische Erfordernisse umfassen. Die geometrischen Erfordernisse können Bemessungen, wie z.B. die Höhe, die Breite, der Umfang, die Ausrichtung eines Skelett- und/oder eines Knochenteils sein. Die Parameter können einen Knochen, ein Weichteil, ein Gewebe und/oder ein Organ betreffen.

Bei einer chirurgischen Versorgung durch Einbringen eines Implantats werden zur präoperativen Planung 2D-Patientenbilder, in der Regel Röntgenbilder, gegenüber 3D-Patientenbildern bevorzugt. Bei der Erzeugung eines 3D-Patientenbildes wird der Patient mit einer geringeren Strahlendosis belastet, als bei einer Röntgenaufnahme.

Bei einem 2D-Patientenbild können eine Skalierung sowie die räumliche Tiefe in Blickrichtung der Aufnahme nicht oder nur eingeschränkt dargestellt werden. Dadurch ist es nur schwer zu beurteilen, ob ein Implantat richtig in oder an dem Knochen des Patienten anliegt. Es ist mit herkömmlichen Mitteln nur schwer zu beurteilen, ob ein Befestigungsmittel für das Implantat, z. B. eine Schraube, richtig in den Knochen eingreift oder ob sie über den Knochen hinausragt. Weiterhin bleibt oftmals unklar, ob die Schraube in den angrenzenden Knochen und/oder in das anliegende Gewebe und/oder in angrenzende Weichteile eindringen könnte.

Wenn ein Koordinatenbezug zwischen einem 2D-Patientenbild und einem 3D-Patientenbild hergestellt wird, können die jeweiligen Bearbeitungsschritte des Planers/Operateurs koordinatenbezogen in die anderen Darstellungen übernommen werden. Hierdurch sind die Bearbeitungsschritte in sämtlichen ausgewählten Darstellungen planbar und prüfbar. Die Darstellungen umfassen insbesondere Belastungs- und Entlastungslagen des jeweiligen Körperabschnitts. Die Darstellungen umfassen zugehörige Dimensionen, vorzugsweise in einer Gegenüberstellung von Sollwerten und Istwerten.

Das erfindungsgemäße Verfahren betrifft auch Arbeiten und Planungsschritte, die der Planer/Operateur in der Arbeitsdarstellung, insbesondere im Zusammenhang mit einem ermittelten möglichen Implantat, durchführt. Die Arbeiten und Planungsschritte umfassen vorzugsweise, aber nicht ausschließlich, die Positionierung eines möglichen Implantats anhand eines dargestellten Implantat-Modells. Die Positionierung kann an einem Wirbel oder an der Wirbelsäule die Veränderung der Form und/oder der Größe eines Implantat-Modells anzeigen. Die Verdrehung oder die Verschiebung des Implantat-Modells kann dargestellt werden. Ebenso kann die Veränderung des Eintrittswinkels eines Implantat-Modells beispielsweise zu einem Wirbel gezeigt werden. Bemaßungen oder Messergebnisse können zeitgleich und im Verhältnis zu den räumlichen Koordinaten auf einem oder mehreren Bildschirmfenstern dargestellt werden.

Die präoperative Planung und/oder die Simulation eines operativen Eingriffs kann in einer oder in mehreren Darstellungen des Körperabschnitts des Patienten erfolgen, an dem der Eingriff durchgeführt wird. Die Darstellungen sind als Arbeitsdarstellungen ausgebildet, in denen wenigstens ein im Verhältnis zum Körperabschnitt bewegbares Modell eines Implantats angeordnet werden kann. Auf die Simulation wird weiter unten gesondert eingegangen.

Die Erfindung versteht unter dem Begriff "Operation am menschlichen Körper" einen vorzugsweise instrumentellen, chirurgischen Eingriff, der am oder im Körper eines Patienten ausgeführt wird.

Die Operation wird vorzugsweise zum Zweck der Therapie durchgeführt. Sie kann auch zum Zweck einer weiteren Diagnostik ausgeführt werden.

Die Operation kann vorzugsweise, aber nicht ausschließlich, ein gynäkologischer Eingriff sein. Es kann sich um eine Geburtshilfe handeln. Die Operation kann im Bereich der Urologie oder im Bereich der Hals-Nasen-Ohren-Heilkunde stattfinden.

Die Operation am menschlichen Körper, in der Folge vereinfachend Operation genannt, umfasst die präoperative Phase, die intraoperative Phase sowie die postoperative Phase.

### Menschlicher oder tierischer Körper

Das vorliegende Verfahren und das dazugehörige System sind auf alle Bereiche des menschlichen und/oder tierischen Körpers anwendbar. Lediglich aus Vereinfachungsgründen wird im Folgenden nur noch von einem menschlichen Körper oder vereinfachend vom Patienten gesprochen.

Das Verfahren sowie das zugehörige System betreffen sämtliche Aspekte und Bestandteile des menschlichen Körpers. Dies sind beispielhaft und in keiner Weise ausschließlich der Kopf, die Haut, die Extremitäten sowie die Organe und das Skelett des Menschen.

Im Weiteren werden das Verfahren sowie das zugehörige System am Beispiel eines Knochens des Skeletts beschrieben.

### Einrichtung zur Durchführung der Operation am menschlichen Körper

Eine Einrichtung zur Durchführung der Operation am Patienten kann ein Krankenhaus oder eine Klinik sein. Zusätzlich kann eine Reha-Klinik oder eine Poliklinik gemeint sein. Bei der Einrichtung kann es sich um ein Klinikum oder eine sonstige medizinische Einrichtung handeln.

Es versteht sich von selbst, dass auch jede andere Einrichtung zur Durchführung von Operationen am menschlichen Körper gemeint sein kann.

In der Einrichtung werden ärztliche und pflegerische Hilfeleistungen an einem Patienten erbracht.

In dem Krankenhaus werden die Krankheiten und/oder Leiden und/oder körperlichen Schäden des Patienten festgestellt. Es wird ein Plan zur Behandlung der Krankheiten, Leiden oder körperlichen Schäden sowie zur Durchführung der Operation ausgearbeitet.

Die Einrichtung zur Durchführung von Operationen am menschlichen Körper kann sich auch auf die Geburtshilfe oder die Sterbebegleitung beziehen.

Die Einrichtung zur Durchführung von Operationen am menschlichen Körper kann sich auf Maßnahmen der Notfallbehandlung beziehen. Darüber hinaus kann eine vollstationäre oder teilstationäre Einrichtung vorliegen. In der Einrichtung zur Durchführung von Operationen am menschlichen Körper können vorstationäre und/oder nachstationäre Behandlungen erfolgen.

Ebenso können ambulante Behandlungsmaßnahmen oder rehabilitative Behandlungsmaßnahmen erfolgen.

Die vorausgehende sowie alle nachfolgenden Aufzählungen sind lediglich beispielhaft gemeint und in keiner Weise abschließend gedacht.

### Visualisierungsmittel

Die Erfindung versteht unter dem Begriff ʺVisualisierung" das Sichtbarmachen von vorzugsweise abstrakten Daten, Texten und/oder Zusammenhängen in einer graphischen und/oder einer visuell erfassbaren Form.

Zur Darstellung der Daten, Inhalte, Texte und/oder Zusammenhänge in der graphischen bzw. visuell erfassbaren Form ist ein Visualisierungsmittel vorgesehen.

Das Visualisierungsmittel ist als eine optische Einrichtung ausgebildet. Das Visualisierungsmittel kann ein Smartphone sein. Es kann auch als Brille ausgebildet sein.

Rein beispielhaft, aber nicht ausschließlich, ist das Visualisierungsmittel zur Kombinierung der natürlichen Wahrnehmung des Bereichs des Körpers durch den Benutzer mit wenigstens einer künstlich erzeugten Darstellung als Brille ausgebildet. Die Brille funktioniert auf der Basis der Augmented-Reality.

Das Visualisierungsmittel kann auch als eine optische Einrichtung ausgebildet sein und an einem Körperteil der Person angeordnet sein, die an der Operation teilnimmt. Vorzugsweise ist die optische Einrichtung am Kopf des Arztes angeordnet.

Die optische Einrichtung ist schwenkbar ausgebildet. Sie kann aus einer Ruhestellung im Bereich des Kopfes in eine Arbeitsstellung verschwenkt werden, in der die Einrichtung wenigstens vor einem Auge des Arztes positioniert ist.

Die Einrichtung ist in einem Abstand vor dem wenigstens einen Auge des Arztes angeordnet, sodass dem Arzt die abstrakten Daten, Texte und sonstigen Zusammenhänge graphisch und/oder visuell erfassbar dargestellt werden können.

Das Visualisierungsmittel umfasst ein Speichermedium zur Abspeicherung der Daten, Werte und Informationen.

Die Daten werden dem Visualisierungsmittel vorzugsweise drahtlos und/oder über eine WLAN-Verbindung aus dem Informationssystem des Krankenhauses übermittelt.

Das Visualisierungsmittel umfasst weiter eine Rechnereinheit, mit der die Daten, Werte und Informationen bearbeitet werden können.

Es versteht sich von selbst, dass die Werte, Informationen und Daten in der Rechnereinheit auch verarbeitet werden können, um sie im Visualisierungsmittel optisch darstellen zu können.

Auf die Rechnereinheit des Visualisierungsmittels wird weiter unten im Detail eingegangen.

Zur Herstellung einer Kommunikation zwischen dem Visualisierungsmittel und dem Informationssystem des Krankenhauses umfasst das Visualisierungsmittel wenigstens eine Schnittstelle.

Über die Schnittstelle kann auch eine Kommunikation zwischen dem Visualisierungsmittel und einem Gerät aufgebaut werden, das vorzugsweise im Krankenzimmer steht. Das Gerät dient zur Überwachung des Patienten oder zur Durchführung weiterer Therapiemaßnahmen. Es versteht sich von selbst, dass das Gerät auch für andere Aufgaben und Funktionen eingesetzt werden kann.

Das Visualisierungsmittel wird wenigstens einer Person zugeordnet, vorzugsweise dem Operateur.

Es versteht sich von selbst, dass das Visualisierungsmittel auch mit Hilfe einer APP funktionieren kann, die vorzugsweise, aber nicht ausschließlich, in das Handy oder ein Tablet oder ein sonstiges elektronisches Gerät eingespielt wurde.

Die Zuordnung des Visualisierungsmittels kann über einen Strichcode erfolgen. Sie kann auch über einen QR-Code erfolgen. Selbstverständlich kann die Zuordnung des Visualisierungsmittels zum Arzt oder zu einer anderen, an der Operation teilnehmenden Person über das Einscannen eines Fingerabdrucks erfolgen. Die Zuordnung des Visualisierungsmittels vorzugsweise zu dem Operateur kann über eine Iriserkennung erfolgen. Hierbei wird mit einer Kamera die Iris des Auges vorzugsweise des behandelnden Arztes erkannt und auf diese Weise das Visualisierungsmittel dem jeweiligen Arzt zugeordnet.

Das Visualisierungsmittel umfasst die Kamera auch zum Zweck der Kombinierung der natürlichen Wahrnehmung des Bereichs des Körpers durch den Benutzer mit wenigstens einer künstlich erzeugten Darstellung.

Es versteht sich von selbst, dass die Zuordnung des Visualisierungsmittels zu einer bestimmten Person auch über jedes andere technische Verfahren möglich ist.

In das Visualisierungsmittel kann eine worklist eingespielt werden, die dem jeweiligen Träger des Visualisierungsmittels die Anzahl, die Namen und/oder die Stationen und Nummern des Operationssaals der Patienten visuell darstellt, die der Arzt am jeweiligen Tag zur Durchführung der Operation besuchen muss.

In der Worklist sind darüber hinaus noch die sonstigen Arbeitsaufgaben und Termine optisch dargestellt, die der Arzt am jeweiligen Tag durchzuführen hat.

Die Worklist kann dem behandelnden Arzt eine Reihenfolge vorgeben, nachdem die Operationen jeweils durchzuführen sind. Die Worklist kann ergänzende Punkte enthalten, die bei der Durchführung der Operation beim jeweiligen Patienten zu beachten sind.

Die Worklist wird mit Daten, Informationen und Werten erstellt, die aus dem Informationssystem des Krankenhauses zur Verfügung gestellt werden.

### Rechnereinheit des Visualisierungsmittels

Das Visualisierungsmittel umfasst eine Rechnereinheit. Die Rechnereinheit dient der Bearbeitung und/oder der Verarbeitung der in dem Speichermedium des Visualisierungsmittels abgespeicherten Daten, Werte und Informationen.

Das Visualisierungsmittel umfasst zur Kombinierung der natürlichen Wahrnehmung des Bereichs des Körpers durch den Benutzer mit wenigstens einer künstlich erzeugten Darstellung eine Rechnereinheit.

In der Rechnereinheit sind sämtliche Schnittstellen des Visualisierungsmittels mit dem Informationssystem des Krankenhauses hinterlegt, über die die Daten aus dem Informationssystem des Krankenhauses in das Speichermedium des Visualisierungsmittels übertragen werden können.

In der Rechnereinheit werden sämtliche Schnittstellen des Visualisierungsmittels zu den Geräten, an die der Patient zur Überwachung des Gesundheitszustandes während der Operation und zur weiteren Behandlung angeschlossen ist, vereinigt und verwaltet. In der Rechnereinheit wird auch die wenigstens eine Schnittstelle vereinigt und verwaltet, die zur Übertragung von Daten, Werten und Informationen an das Speichermedium des Visualisierungsmittels aus dem Informationssystem des Krankenhauses vorgesehen ist.

Auf diese Weise können zur Durchführung der Operation sämtliche Daten, Werte und Informationen der Geräte, an die der Patient angeschlossen ist, zugeordnet und/oder ausgewertet und/oder miteinander verglichen werden.

Auf diese Weise kommuniziert die Rechnereinheit des Visualisierungsmittels mit dem wenigstens einen Gerät, an das der Patient im Krankenhaus zur Durchführung der Operation angeschlossen ist.

Die Rechnereinheit des Visualisierungsmittels ist zur automatischen Übertragung von Daten, Werten und Informationen während der Operation aus dem Informationssystem des Krankenhauses ausgebildet.

Die automatische Übertragung der Daten, Werte und/oder Informationen erfolgt in Abhängigkeit des jeweiligen Operationssaals, in dem sich der behandelnde Arzt mit seinem Visualisierungsmittel gerade aufhält.

Die automatische Übertragung der Daten aus dem Informationssystem des Krankenhauses an die Rechnereinheit des Visualisierungsmittels kann auch in Abhängigkeit der jeweiligen Uhrzeit erfolgen.

Sie kann auch in Abhängigkeit davon erfolgen, inwieweit der behandelnde Arzt und/oder Träger des Visualisierungsmittels die ihm überspielte Worklist für die jeweils durchzuführenden Operationen bereits abgearbeitet hat.

Es versteht sich von selbst, dass der Träger des Visualisierungsmittels über das Visualisierungsmittel Daten manuell und/oder über eine andere technische Art und Weise abrufen kann.

Die automatische Übermittlung der Daten in das Speichermedium des Visualisierungsmittels erfolgt vor, während und nach der Operation, vorzugsweise dann, wenn sich der behandelnde Arzt mit seinem Visualisierungsmittel dem Operationsaal nähert, in dem der Patienten zur Operation vorbereitet ist.

Die automatische Einspielung von Daten, Werten und Informationen zu dem Patienten, dessen Operation folgt, kann dann durchgeführt werden, wenn die Operation des vorherigen Patienten beendet ist und der behandelnde Arzt den Operationsaal wieder verlässt.

Die automatische Übermittlung von Daten, Werten und Informationen kann sich sowohl auf Daten aus dem Informationssystem des Krankenhauses beziehen, als auch Daten umfassen, die von wenigstens einem Gerät ausgesendet werden, an das der Patient im Krankenhaus zur Überwachung während der Operation und/oder zur weiteren Behandlung nach der Operation angeschlossen ist.

### Natürliche Wahrnehmung

Unter dem Begriff "natürliche Wahrnehmung" versteht die Erfindung, dass vorzugsweise der Operateur seine Umwelt in sämtlichen Eigenschaften mit den ihm zur Verfügung stehenden Sinnen erfährt.

Der Operateur ist dabei in der Lage, sämtliche Ereignisse und Dinge zu erkennen und zu klassifizieren. Auf diese Weise kann sich der Operateur auf Reaktionen aus seiner direkten Umgebung vorbereiten.

Vor, während und nach der Operation konzentriert der Operateur seine optische und/oder natürliche Wahrnehmung auf den Patienten. Er richtet seine optische und/oder natürliche Wahrnehmung auch auf Bereiche des Körpers des Patienten, die der Operation unterzogen werden sollen.

Die natürliche und/oder visuelle Wahrnehmung, vorzugsweise durch den Operateur, basiert auf Lichtreizen, die auf die Netzhaut des Auges des Operateurs einfallen.

### Erzeugung der künstlichen Darstellung mit Hilfe eines Visualisierungsmittels auf Basis einer "virtual Reality" und/oder einer "mixed Reality" und/oder einer "augmented Reality"

Die Erzeugung der künstlichen Darstellung erfolgt mit Hilfe eines Visualisierungsmittels, vorzugsweise, aber nicht ausschließlich, auf Basis einer "virtual Reality" und/oder einer "mixed Reality" und/oder einer "augmented Reality.

Durch die Einblendung von Daten, Werten und Informationen in das Visualisierungsmittel in Form einer augmented Reality nimmt der Träger des Visualisierungsmittels die von ihm aufgerufenen Daten, Werte und Informationen als "vor sich im Raum schwebende Projektionen" wahr.

Rein beispielhaft, aber nicht ausschließlich, wird im Weiteren davon ausgegangen, dass die Erzeugung der künstlichen Darstellung auf Basis einer "mixed Reality" erfolgt.

Durch die Einblendung von Daten, Werten und Informationen in das Visualisierungsmittel in Form einer mixed Reality nimmt der Träger des Visualisierungsmittels die von ihm aufgerufenen Daten, Werte und Informationen als "vor sich im Raum schwebende Projektionen" wahr

Unter dem Begriff "virtual Reality" versteht die Erfindung die Darstellung und gleichzeitig die Wahrnehmung z.B. eines Menschen in einer computergenerierten, interaktiven virtuellen Umgebung, die in Echtzeit generiert wird.

Der Begriff "mixed Reality" bezeichnet die Wahrnehmung eines Menschen von seiner natürlichen Umgebung. In die Wahrnehmung der natürlichen Umgebung des Menschen werden vorzugsweise computererzeugte, virtuelle Realitäten eingeblendet.

Die Erfindung bezeichnet unter dem Begriff "augmented Reality" eine erweiterte Realität, die durch eine computergestützte Erweiterung der Wahrnehmung des Nutzers erreicht wird.

Mit anderen Worten bezeichnet die augmented Reality vorzugsweise eine visuelle Darstellung von Informationen, wodurch Bilder oder auch Videos mit Computer generierten Zusatzinformationen ergänzt werden.

Im Weiteren wird davon ausgegangen, dass das Visualisierungsmittel auf Basis einer mixed Reality funktioniert.

Die Erfindung sieht vor, dass die Daten, Werte und Informationen in dem Visualisierungsmittel in Form einer mixed Reality dargestellt werden.

Durch die Einblendung von Daten, Werten und Informationen in das Visualisierungsmittel in Form einer mixed Reality nimmt der Träger des Visualisierungsmittels, vorzugsweise der Operateur die von ihm aufgerufenen Daten, Werte und Informationen als "vor sich im Raum schwebende Projektionen" wahr.

Der Träger des Visualisierungsmittels kann aufgrund der mixed Reality die Informationen, die "vor ihm im Raum schweben" verschieben, erweitern und/oder verändern.

Mit Hilfe der mixed Reality kann der Träger des Visualisierungsmittels in einem "vor sich schwebenden Buch", das mit Hilfe der mixed Reality in sein Visualisierungsmittel eingeblendet wird, z.B. Seiten umblättern.

Er kann Passagen und Darstellungen des virtuellen Buches extrahieren und gesondert im Speichermedium des Visualisierungsmittels abspeichern.

Mit Hilfe der mixed Reality kann sich der Träger des Visualisierungsmittels, vorzugsweise der Operateur, Daten, Werte und Informationen in sein Visualisierungsmittel einblenden lassen. Die eingeblendeten Daten, Werte und Informationen stehen der Person, die die Operation durchführt, als Zusatzinformation und/oder als Vergleichswerte bei der Operation des Patienten und/oder zur Beurteilung des Verlaufs der Operation des Patienten zur Verfügung.

Zur Erstellung der Dokumentation über die Operation lässt sich der behandelnde Arzt den von ihm diktierten Bericht in Form einer mixed Reality-Darstellung in sein Visualisierungsmittel einblenden.

Der behandelnde Operateur sieht dabei das von ihm erstellte Diktat in Form eines Textes, der "vor seinem Visualisierungsmittel im Raum schwebt".

Der Operateur kann Fotos und Abbildungen, die er mit Hilfe des Visualisierungsmittels vor oder während der Operation erstellt hat, während der Operation vergleichen und in seine Dokumentation einfügen.

Die Funktion des Visualisierungsmittels auf Basis der mixed Reality ermöglicht es, dass der behandelnde Arzt die Dokumentation der von ihm durchgeführten Visite am Krankenbett des Patienten erledigen kann.

Dadurch, dass das Visualisierungsmittel auf Basis der mixed Reality funktioniert, sieht der Operateur seinen Bericht zur Operation in Textform umgesetzt "vor ihm im Raum schwebend".

Er ist dadurch in der Lage, den Bericht in zeitlich engem Zusammenhang mit der Operation oder noch während der Operation auf Korrektheit und Vollständigkeit zu überprüfen. Die Dokumentation der von ihm durchgeführten Operation kann z.B. an die anderen Teilnehmer seines Operationsteams und/oder vorzugsweise an das Informationssystem des Krankenhauses weitergeleitet werden.

### Bildassistenzeinrichtung

Die Bildassistenzeinrichtung im Sinne dieser Erfindung ist in der Lage, dem Operateur zugängliche Datenbestände zu visualisieren, zu skalieren und zu lesen. Mindestens zwei oder mehrdimensionale Darstellungen, zumindest eines Abschnitts des Patientenkörpers, können auf die natürliche Wahrnehmung des Abschnitts des Patientenkörpers projiziert werden.

Die Bildassistenzeinrichtung ist eine bevorzugt mit elektronischen Mitteln und zugehöriger Software arbeitende Einrichtung, die wenigstens eine Schnittstelle aufweist. Die Bildassistenzeinrichtung ermöglicht es, Bilddaten, anatomische Daten und geometrische Daten bei Erfordernis von wenigstens einem Datenspeicher abzurufen. Der Datenspeicher arbeitet mit bildgebenden Verfahren und/oder mit nicht an die Einrichtung angebundenen Geräten oder Einrichtungen. Daten können durch Handeingabe empfangen oder verarbeitet werden. Sie können in einem softwaregebundenen Algorithmus derart aufbereitet werden, dass eine präoperative Planung und/oder eine Ausgabe von Planungsdaten an interoperativ benutzte Anzeige-, Hilfs-, Mess- oder bildgebende Aufnahmeeinrichtungen übermittelt werden kann. Die Daten können von diesen interoperativ abgerufen oder in Echtzeit verarbeitet und an die angeschlossenen Einrichtungen zurücksendet werden.

Bildgebendes Verfahren ist jedes prä- oder interoperativ genutzte Verfahren, durch das Informationen über den Aufbau eines menschlichen oder tierischen Körpers oder Teilen davon oder dessen Eigenschaft und dessen aufgenommene Bilder zum Zweck präoperativer Planung und/oder interoperativer Kontrolle durch die Bildassistenzeinrichtung übernommen, ausgewertet, verarbeitet und über eine Schnittstelle an angeschlossene Anzeige-, Hilfs-, Mess- oder bildgebende Einrichtungen übermittelt werden können.

Schnittstelle ist eine Einrichtung, die unter Anwendung elektrischer, funktechnischer, lichttechnischer oder magnetischer Übertragungsverfahren auch unter Einbeziehung von programmtechnischen Algorithmen in der Lage ist, von der Bildassistenzeinrichtung generierte Daten an Anzeige-, Hilfs-, Mess-, bildgebende Aufnahme- oder Navigationseinrichtungen zu übermitteln und/oder Informationen von Anzeige-, Hilfs-, Mess- oder bildgebenden Aufnahmeeinrichtungen zu empfangen.

Navigationssystem ist eine Einrichtung zur Unterstützung von Operationen am menschlichen oder tierischen Körper. Sie dient bevorzugt der Einstellung erforderlicher geometrischer und räumlicher Verhältnisse. Sie kann die tatsächlichen geometrischen Verhältnisse erfassen und ist in der Lage, über die Schnittstelle der Bildassistenzeinrichtung sowohl Informationen von dieser zu empfangen als auch an diese zu liefern.

Steuerung ist das über das präoperative Planungsverfahren hinausgehende Abrufen von durch die Bildassistenzeinrichtung generierten Bildinformationen durch Handeingabe, Gesten oder Informationen angeschlossener bildgebender Einrichtungen oder Informationen angeschlossener Einrichtungen zur Erfassung geometrischer Daten.

Weiterhin ist es ein Ziel der Erfindung, eine Skalierung der Informationen bei den Patientenbildern vornehmen zu können.

Erfindungsgemäß kann die präoperative Situation unter anderem durch eine automatische, halbautomatische oder manuelle Berechnung aller relevanten Winkel und sonstigen anatomischen oder operativen Parametern, etwa der Materialbeschaffenheit, der Größe etc., eines möglichen einzusetzenden Implantats und der Patientendaten analysiert und dem Verfahren zu Grunde gelegt werden.

Das Verfahren zur Planung, Vorbereitung, Überwachung und Begleitung eines operativen Eingriffs wird an einer Bildassistenzeinrichtung durchgeführt, die dem Planer/Operateur eine archivierbare, speicherbare und reproduzierbare präoperative Planung ermöglicht und hierdurch seine Arbeit erleichtert.

Die Bildassistenzeinrichtung im Sinne dieser Erfindung ist in der Lage, dem Planer/Operateur zugängliche Datenbestände zu visualisieren, zu skalieren und zu lesen, mindestens zwei mehrdimensionale Darstellungen zumindest eines Abschnitts des Patientenkörpers zu zeigen, von denen sich wenigstens zwei Darstellungen wenigstens in Bezug auf die Anzahl der Dimensionen unterscheiden.

Die Bildassistenzeinrichtung erkennt die zugänglich gemachten Patientenbilder unter anderem in Bezug auf ihr Format z.B. als 3D-Bild und/oder 3D-Bilddaten bzw. als 2D-Bild und/oder 2D-Bilddaten.

Die Informationen können der Bildassistenzeinrichtung beispielsweise elektronisch, über das Internet, unter Verwendung geeigneter Übertragungsprotokolle und/oder auf anderem Wege, insbesondere für den Abruf, zum Einspielen bzw. Herunterladen und Abbilden, zugänglich gemacht werden.

Die Datenübertragung kann direkt von der Festplatte eines Computers erfolgen oder mittels eines anderen mobilen oder feststehenden Datenträgers einer Festplatte, einer CD, einer DVD, einer Flash-Speichervorrichtung (z. B. Speicherstift, Compact-Flash, sichere Digitalkarte), einem sog. Cloud-System.

Ferner sind verschlüsselte oder unverschlüsselte Übermittlungen per E-Mail oder über andere digitale Übermittlungen zu jedem geeigneten Typ von Computervorrichtung, Smartphone, PDA, Tabletcomputer oder zu anderen Vorrichtungen möglich, über die elektronische Informationen übermittelt werden können.

Es ist auch denkbar, der Bildassistenzeinrichtung aus einem 3D-Patientenbild gewonnene 2D-Schichten oder bereits vorhandene externe 2D-Schichten von 3D-Patientenbildern zugänglich zu machen und für die weitere Bearbeitung und/oder Planung des operativen Eingriffs sowie des Einsatzes eines orthopädischen Implantats zur Verfügung zu stellen. Gleiches gilt entsprechend für aus 2D-Patientenbildern gewonnene 3D-Bilder.

Die Bildassistenzeinrichtung kann zudem 2D-Schichten aus einem 3D-Patientenbild ableiten und errechnen und umgekehrt. Zur Darstellung der 2D-Schicht wird in den dreidimensionalen Körper, beispielsweise Knochen, Gewebe, Gelenke oder Weichteile, insbesondere den Wirbeln der Wirbelsäule, eine Ebene gelegt, die horizontal oder vertikal oder in einem Winkel zur Achse der Wirbelsäule verlaufen kann. Die Koordinaten der Ebene bestimmen die Koordinaten der 2D-Schicht und ermöglichen es, die Perspektive einer bestimmten zweidimensionalen Schicht des dreidimensionalen Körpers darzustellen. Die Berechnung der 2D-Daten kann automatisch erfolgen.

Ferner sind die 2D- und die 3D-Patientenbilder, die dem Verfahren zur Verfügung stehen, mit Hilfe der Bildassistenzeinrichtung in einer hochauflösenden MIP- bzw. einer MPR-Darstellung anzeigbar.

Ferner können erfindungsgemäß mit Hilfe der Bildassistenzeinrichtung 2D-Patientenbilder vor, während oder nach deren Zugänglichmachung bearbeitet, insbesondere skaliert, werden. Dabei werden automatisch, halbautomatisch oder manuell Abmessungen des abgebildeten Körperabschnitts des Patienten, insbesondere der Wirbelsäule, und/oder der Maßstab der Aufnahme mit Hilfe der Bildassistenzeinrichtung ermittelt, beispielsweise durch verwenden eines bekannten Referenzobjekts, etwa einer Kugel aus einem nicht strahlendurchlässigen Material.

Der Skalierungsvorgang ist für jedes einzelne überspielte 2D-Patientenbild gesondert durchführbar und wiederholbar.

Die Bildassistenzeinrichtung ermöglicht es dem Operateur ferner, die zugänglich gemachten 3D-Patientenbilder in unterschiedlichen Ansichten, Ausschnitten und/oder Blickwinkeln zu segmentieren oder zu skalieren. Dies ermöglicht die Volumenerkennung und nicht nur die Erkennung einzelner Punkte der Patientenbilder. Die Segmentierung kann auch in einem Netz oder in einem Cloud-System erfolgen und gespeichert werden.

Die Segmentierung ermöglicht die Erkennung und medizinische/anatomische Bezeichnung und/oder Bezifferung einzelner Knochen, Wirbel, Gelenke, Weichteile, Gewebe etc. Die Segmentierung ermöglicht weiter die Erkennung und die medizinische/anatomische Bezeichnung und/oder die Bezifferung insbesondere der Wirbel der Wirbelsäule anhand von Referenzmodellen und Soll-Werten. Sie ermöglicht den Vergleich mit medizinischen und anatomischen Ideal- und Normwerten der menschlichen oder tierischen Anatomie, um die durch die Operation angestrebte Patientenanatomie möglichst ideal durchführen zu können, die der Bildassistenzeinrichtung, wie oben dargestellt, zugänglich gemacht werden können. Zudem können mit Hilfe der Bildassistenzeinrichtung einzelne Knochen, Weichteile und Gewebeteile in den Darstellungen ein- oder ausgeblendet oder hervorgehoben werden.

Die Segmentierung mit Hilfe der Bildassistenzeinrichtung, also Erkennung und Bezeichnung insbesondere einzelner Knochen und/oder Skelettteile, Wirbel, Gelenke, Weichteile, Gewebe etc., kann insbesondere automatisch erfolgen und beispielsweise manuell überprüft werden.

Dies kann insbesondere bei Wirbeln anhand von zugänglich gemachten Skizzen, insbesondere Wirbelskizzen, dargestellt, verdeutlicht und/oder überprüft werden.

Erfindungsgemäß können mit Hilfe der Bildassistenzeinrichtung an einem Bildschirm Bildschirmfenster mit unterschiedlichen Darstellungen, beispielsweise unterschiedliche Patientenbilder, Skizzen des operationsrelevanten Körperabschnitts, z.B. einer Wirbelsäule oder eines Wirbels, zeitgleich dargestellt werden.

Dabei ist es mit einem weiteren Aspekt der Erfindung möglich, mit Hilfe der Bildassistenzeinrichtung einen räumlichen Koordinatenbezug zwischen 2D-Patientenbildern und 3D-Patientenbildern herzustellen. Insbesondere kann eine Korrelation zwischen den vorzugsweise räumlichen Informationen von Daten und Informationen in 2D-Patientenbildern und 3D-Patientenbilder hergestellt werden und umgekehrt. Auf diese Weise sind Informationen aus einer Darstellung in eine andere Darstellung übertragbar, anzeigbar und bearbeitbar.

Das erfindungsgemäße Verfahren sieht des Weiteren die Möglichkeit vor, durch den Operateur mit Hilfe der Bildassistenzeinrichtung Patientenbilder, die in wenigstens einem Bildschirmfenster dargestellt werden, zu bearbeiten.

Insbesondere kann der Operateur beispielsweise durch Anklicken etwa mit einem Cursor, eine der Darstellungen in wenigstens einem Bildschirmfenster zur Bearbeitung in einer Arbeitsdarstellung aktivieren.

Unter Cursor in diesem Sinne ist jede Anzeige-, Eingabe-, Befehlsgebe- und/oder Bearbeitungsvorrichtung zu verstehen. Beispielsweise ist darunter eine Maus, ein Trackball, eine Tastatur, die Anzeige eines Touchscreens oder eines Grafiktabletts, insbesondere mit einem Zeiger, zu verstehen.

Dabei kann mindestens eine der mehrdimensionalen Darstellungen, insbesondere wenigstens eine 2D-Darstellung oder eine 3D-Darstellung, mit Hilfe der Bildassistenzeinrichtung als Arbeitsdarstellung aktiviert werden, wobei ein räumlicher Koordinatenbezug zwischen den einzelnen Darstellungen herstellbar ist.

Eine mit der 3D-Darstellung korrespondierende 2D-Darstellung kann ebenfalls mittels des Cursors bearbeitet werden. Dabei können 2D-Darstellungen und/oder 2D-Schichten z.B. mit Hilfe des Scrollrades einer Maus oder eines ähnlichen rechnerverbundenen Zeigemechanismus durchlaufen werden. Die jeweilige Arbeitsposition wird zeitgleich und in den entsprechenden räumlichen Koordinaten auf der jeweiligen Darstellung in jedes andere Bildschirmfenster übertragen. Das erfindungsgemäße Verfahren ermöglicht so eine genaue Orientierung und Übertragung von Koordinaten sowohl in 2D-Darstellungen als auch in korrespondierenden 3D-Darstellungen eines dargestellten Abschnitts des Patientenkörpers. Der Cursor verdeutlicht, welchen Bereich einer 3D-Darstellung und/oder einer 2D-Darstellung der Planer/Operateur zum jeweiligen Zeitpunkt markiert und/oder beplant und/oder bearbeitet.

Derartige Bearbeitungsmaßnahmen etwa in 3D-Patientenbildern können vorzugsweise automatisch in ihren Auswirkungen auch in 2D-Patientenbildern, vorzugsweise unter Herstellung eines Koordinatenbezuges, wiedergegeben werden. Gleiches gilt umgekehrt, wenn die Bearbeitungsmaßnahmen in 2D-Patientenbildern vorgenommen werden.

Externe Geräte können Bildassistenzeinrichtungen, Anzeige-, Hilfs-, Messgeräte oder vorzugsweise ein mobiles oder ein stationäres (Operations-)Navigationssystem sein. Das Navigationssystem dient insbesondere zur Steuerung und Überwachung eines operativen Eingriffs, insbesondere zur Bestimmung der örtlichen Position und/oder zur Bestimmung der Koordinaten des Eingriffs und der Einleitung und der Durchführung von operativen Maßnahmen. Die Planungsdaten können beispielsweise kabellos oder kabelgebunden über geeignete Protokolle übermittelt werden.

Die Schnittstelle kann ferner so konfiguriert sein, dass sie in der Lage ist, entfernt angeordnete Anzeigegeräte mit Informationen zu versorgen. Beispielsweise kann dies ein in einem Operationssaal angeordneter Bildschirm sein.

Die Schnittstelle kann so ausgelegt sein, dass sie nicht nur Daten von externen Quellen übernehmen kann oder nur Steuerdaten an eine Anzeigeeinrichtung liefern kann. Bei entsprechender Ausrüstung der Schnittstelle ist es möglich, permanent Daten von externen Quellen zu laden, diese mit gespeicherten Daten zu vergleichen und über eine Anzeigeeinrichtung dem Operateur zur Verfügung zu stellen. Mögliche Daten sind dabei neben den oben beschriebenen Bilddaten auch durch externe Messeinrichtungen ermittelte geometrische Daten, beispielsweise Daten über Lage, Ausrichtung oder Fortschritt eines operativen Eingriffs.

Dabei ist es unter anderem möglich, sog. elektronische Zäune, beispielsweise zur Begrenzung des Operationsbereichs und/oder zur Markierung von bestimmten Bereichen im Körper des Patienten, zur genauen Positionierung eines Implantats in der mehrdimensionalen, vorzugsweise dreidimensionalen Planung, festzulegen und zur Ein- und/oder Ausgrenzung dieser Bereiche sowie zur örtlichen Koordination des Eingriffs und/oder des Implantats vorzunehmen und die zugrunde liegenden Koordinaten an ein mobiles oder stationäres Operations-Navigationssystem kabellos oder kabelgebunden zu überspielen.

Es ist weiterhin möglich, die bei der präoperativen Planung ermittelten Daten in ein 3D-Modell des Implantats zu übersetzen und diese Daten über die Schnittstelle an mit dieser verbundene Maschinen zu senden. Dies können beispielsweise spanabhebende Werkzeugmaschinen sein, die entweder ein neues Implantat herstellen oder vorhandene Implantate individuell nachbearbeiten. Ebenso ist es möglich, einen 3D-Drucker anzusteuern, der entweder ein angepasstes Implantat herstellt oder ein nicht als Implantat geeignetes Muster erzeugt, das abhängig vom Bedarf Studienzwecken während der präoperativen Planung dient, als Modell für Lehrzwecke geeignet ist oder auch als Modell für ein durch Urformen herzustellendes Implantat dienen kann.

Eine weitere Möglichkeit besteht darin, dass die Ergebnisse der präoperativen Planung über die Schnittstelle einem Navigationssystem übermittelt werden, wobei entweder direkt Steuerdaten übermittelt werden oder das Navigationssystem aus erhaltenen Planungs- oder Bilddaten selbst Steuerdaten gewinnt.

Es ist ebenso möglich, dass die Bildassistenzeinrichtung über seine Schnittstelle nicht nur Daten an angeschlossene Geräte liefert. Es kann ebenso über die Schnittstelle eine Rückübertragung von Daten der externen Geräte an die Bildassistenzeinrichtung durchgeführt werden. Damit wird es möglich, durch ständigen Vergleich der Soll-, Planungs- und Ist-Daten während der Operation eintretende Veränderungen festzustellen, um faktisch ein Monitoring der durchgeführten Operation zu ermöglichen.

Neben der automatischen Erfassung von Daten und dem automatischen Abgleich ist des Weiteren eine zusätzliche visuelle Überwachung der Vorgänge im zu operierenden Körperteil beziehungsweise im Körper zu erkennen, die der Operateur möglicherweise nicht erkennen kann.

Kann die Bildassistenzeinrichtung oder die Schnittstelle ein Monitoring ausführen oder ermöglichen, wird dadurch gewährleistet, über die Schnittstelle auch Daten oder Befehle an angeschlossene externe Geräte zu übermitteln. Insbesondere bei der Verwendung eines Navigationssystems besteht so die Möglichkeit, auf Abweichungen von geplanten Maßnahmen und dem Planungsergebnis aufmerksam zu machen und somit Unregelmäßigkeiten bei der Durchführung der Operation zu vermeiden. Die Art der gegebenen Hinweise kann von den Fähigkeiten des Navigationsgerätes abhängen. Es kann beispielsweise eine optische und/oder akustische Information ausgegeben werden.

Die Bildassistenzeinrichtung kann über die Schnittstelle Bilddaten an einen Monitor im Operationssaal ausgeben. In Fällen, in denen der Operateur Details der präoperativen Planung oder aktuelle Veränderungen nachvollziehen muss, kann über die Schnittstelle eine Gestensteuerung an die Bildassistenzeinrichtung angekoppelt werden. So besteht die Möglichkeit, trotz vorhandener hygienischer Bedingungen im Operationssaal zeitnah nachzuprüfen, ob die Operation mit ihren Ergebnissen den Vorgaben des Planungsergebnisses entspricht.

Dieses Planungsergebnis kann über die Schnittstelle der Bildassistenzeinrichtung mit Patientenbildern, insbesondere 2D- und/oder 3D-Patientenbildern, korreliert werden. Dabei wird eine oder mehrere Röntgen-, CT-, MRT- oder andere in einem bildgebenden Verfahren hergestellte Aufnahme des Patienten in der Lage auf dem Operationstisch erstellt.

Zusätzlich besteht die Möglichkeit, die Aufnahme mit bekannten Verfahren zu kalibrieren. Mit Hilfe der Schnittstelle der Bildassistenzeinrichtung wird eine Korrelation der Koordinaten der mehreren Patientenbilder hergestellt, so dass das Patientenbild des Patienten auf dem Operationstisch in Koordination, Ausrichtung und Darstellung mit der Darstellung des Planungsergebnisses in der Bildassistenzeinrichtung, insbesondere der Navigationsansicht, übereinstimmt.

Über die Schnittstelle der Bildassistenzeinrichtung und des Navigationssystems ist es zudem möglich, das Planungsergebnis und/oder die Teile des Planungsergebnisses und/oder das mindestens auf dem Operationstisch von dem Patienten erstellte Patientenbild einer bildgebenden Vorrichtung, beispielsweise einem Röntgengerät, einem MRT-Gerät oder einem CT-Gerät zur Verfügung zu stellen. Zudem ist es über die Schnittstelle der Bildassistenzeinrichtung und des Navigationssystems möglich, der Bildassistenzeinrichtung und dem Navigationssystems Daten und Informationen zur Verfügung zu stellen, zu speichern, anzuzeigen und darzustellen.

Erfindungsgemäß kann das Planungsergebnis und/oder Teile des Planungsergebnisses und/oder Patientenbilder, die von den Patienten auf dem OP-Tisch angefertigt wurden, an eine bildüberwachende Vorrichtung kabelgebunden oder kabellos übermittelt werden. Die bildüberwachende Vorrichtung ermittelt nach bekannten Verfahren virtuell Lage und Position des Patienten sowie die Lage, die Position und den Fortschritt von einzelnen Schritten der geplanten Operation.

Auf Grund der Kalibrierung sind die tatsächlichen Abmessungen und Dimensionen des Patienten mittels der Bildassistenzeinrichtung gespeichert und können dem Navigationssystem über eine Schnittstelle übermittelt werden.

Instrumente oder Implantate sind beispielsweise aus der verfahrensgemäßen Planung ebenfalls mittels der Bildassistenzvorrichtung gespeichert oder werden mit Hilfe der Bildassistenzeinrichtung zugänglich gemacht, beispielsweise unter Zugriff auf bekannte gespeicherte Informationen oder durch bildgebende und kalibrierte Aufnahmen der Instrumente oder Implantate.

Die Bildassistenzeinrichtung und das Navigationssystem ermöglichen eine automatische Korrelation der Position, Ausdehnung und der Lage des Patienten mit der Position, Ausrichtung und Lage von Implantaten oder Instrumenten.

Zusätzlich können die Instrumente oder Implantate mit wenigstens einem Ortungsmittel verbunden sein, welche die relative Position der Instrumente oder Implantate und Veränderungen in Position, Ausdehnung und Lage der Instrumente oder Apparate an die Bildassistenzeinrichtung und das Navigationssystem übermitteln können.

Mit Hilfe der Bildassistenzeinrichtung und des Navigationssystems können alle Werkzeuge, Implantate und die Lage des Patienten relativ zueinander so dargestellt werden, wie sie auch tatsächlich im Raum liegen.

Insbesondere werden die Führungslinien der geplanten Eingriffe und der geplanten Implantate sowie elektronische Zäune mit Hilfe des Navigationssystems oder der Bildassistenzeinrichtung angezeigt. Dem Operateur ist es somit möglich, in einem Echt-Zeit-Vergleich die einzelnen Operationsschritte an die präoperativ geplanten Operationsschritte anzupassen. Der Operateur kann erfindungsgemäß insbesondere Lage, Position, Eintrittswinkel, Tiefe, Positionierung und Durchführung der präoperativ geplanten Schritte an das Planungsergebnis anpassen. Der Operateur kann hierdurch verhindern, beispielsweise zu tief und/oder in einem falschen Winkel oder an der falschen Stelle Operationsschritte vorzunehmen.

Erfindungsgemäß wird zudem eine Vorrichtung zur Ausführung eines operativen Eingriffs nach dem erfindungsgemäß erlangten Planungsergebnis eines operativen Eingriffs und/oder Teilergebnisse der Planung zur Verfügung gestellt und zur Ausführung eines erfindungsgemäß geplanten operativen Eingriffs verwendet.

Die in die Bildassistenzeinrichtung eingespeisten Daten und/oder Darstellungen werden vorzugsweise bearbeitet und/oder verarbeitet.

Die in die Bildassistenzeinrichtung eingespeisten Daten und/oder Darstellungen umfassen Implantat-Typen und Abmessungen von Implantaten unterschiedlicher Herstellung. Zusätzlich sind von der Bildassistenzeinrichtung von verschiedenen Herstellern angefertigte und auf dem Markt erhältliche Implantat-Varianten umfasst.

### Darstellungen und/oder Daten

Patientenbilder sind unter anderem Röntgenaufnahmen, bei denen es sich um 2D-Aufnahmen bzw. -Bilder handelt, sowie volumenbasierte Aufnahmen, wie beispielsweise CT oder MRT, bei denen es sich um 3D-Aufnahmen bzw. -Bilder handelt.

Volumenbasierte Aufnahmen, also 3D-Aufnahmen bzw. Bilder, bestehen in der Regel aus mehreren Schichten von 2D-Bildern (sog. 2D-Schichten). Diese können in einzelne 2D-Bildschichten unterteilt werden.

Volumenbasierte Bilder werden im Gegensatz zu 2D-Aufnahmen bzw. Bildern bereits bei der Aufnahme skaliert. Dies bedeutet, dass Größe des aufgenommenen Gegenstands und Maßstab der Aufnahme bekannt sind. Die jeweiligen Datenangaben zur Skalierung des Bildes werden gemeinsam mit der jeweiligen volumenbasierten Aufnahme als Datensatz gespeichert.

Demgegenüber müssen 2D-Bilder in der Regel von dem Planer/Operateur zur Planung eines operativen Eingriffs im Vorfeld der Planung skaliert werden, um Maßstab und mögliche Verzerrungen in der Tiefe des dargestellten Abschnitts eines Patientenkörpers festlegen zu können.

Planungsdaten können hier in Form der 2D-Bilddaten vorliegen bzw. aus diesen z. B. durch Bildbetrachtung oder -vermessung gewonnen werden.

Bekannte rechnergestützte Systeme zur präoperativen Planung können dem Planer/Operateur zusätzlich zur Darstellung von Patientenbildern und von möglichen Implantaten auch eine Auswahl eines bestimmten Implantats und dazugehöriger Instrumente, die bei dem chirurgischen Eingriff zu verwenden sind, vorschlagen. Gleiches gilt für Gattungen derartiger Implantate oder Instrumente.

Bei der bekannten Verwendung nur von 2D-Patientenbildern oder nur von 3D-Patientenbildern zur Planung eines operativen Eingriffs besteht jedoch der Nachteil, dass beispielsweise Verschiebungen von Gelenken, Belastungen, Entlastungen oder Verformungen von Knochen und Gelenken, insbesondere pathologische Verformungen der Wirbelsäule, wie beispielsweise Kyphose oder Lordose, oder unterschiedliche Belastungsprofile, nicht in Aufnahmesystemen abbildbar und deshalb nicht in die präoperative Planung einbeziehbar sind.

So werden beispielsweise Volumenpatientenbilder beim liegenden Patienten aufgenommen; hier erfolgt also eine Aufnahme des Patienten häufig in einem entlasteten Zustand.

2D-Patientenbilder, z.B. Röntgenaufnahmen, der Wirbelsäule, werden hingegen häufig bei stehenden Patienten aufgenommen, weil die Aufnahmesysteme diese Lage einfacher darstellen können und der betreffende Körperteil in seinem tatsächlichen Belastungszustand wiedergegeben werden kann.

Insbesondere bei der Planung von Eingriffen an der menschlichen oder tierischen Wirbelsäule, aber auch an deren Gelenken oder Skelettteilen, ist es jedoch notwendig, unterschiedliche Aufnahmesituationen und Lagen des Patienten, insbesondere stehende und liegende Situationen, beispielsweise zur Bestimmung der sagittalen Balance, einbeziehen zu können, zumal der operative Eingriff am liegenden Patienten vorgenommen wird.

Erforderlich ist es deshalb, die sich aus 2D- und 3D-Patientenbildern ergebenden Informationen - insbesondere was den Belastungs- und Entlastungszustand von Skelettteilen, Knochen, Gelenken, Wirbeln, Gewebe, Weichteilen anbelangt - dem Planer des operativen Eingriffs/Operateur auf einfache Weise zur Verfügung zu stellen, so dass er sie vorzugsweise gleichzeitig betrachten und weiterverarbeiten kann.

Patientenbilder sind beispielsweise durch bildgebende Verfahren entstandene zwei- oder dreidimensionale Aufnahmen, die durch bekannte Verfahren zu erhalten sind. Bevorzugt handelt es sich um 2D-Aufnahmen aus der Anwendung von Röntgenverfahren beziehungsweise um 3D-Aufnahmen aus der Anwendung von CT oder MRT. Soweit im Folgenden von 2D- und/oder 3D-Patientenbildern die Rede ist, wird hierunter neben der eigentlichen Abbildung auch der zugrundeliegende Datensatz verstanden.

Soweit nicht anders ausgeführt, werden die Begriffe Information und Daten im Folgenden synonym verwendet.

Die Patientenbilder und Patienteninformationen können beispielsweise zu verschiedenen Zeitpunkten entstanden sein, 2D- und/oder 3D-Bild-Datensätze des Patienten und/oder weitere Daten umfassen, insbesondere zu Körpergröße, Gewicht, Alter des Patienten und etwa Daten aus vorangegangenen Eingriffen, Daten aus vorherigen Untersuchungen und/oder solche Daten umfassen, die in unmittelbarem Zusammenhang mit einer Operation erstellt wurden.

Darüber hinaus können der Bildassistenzeinrichtung zur Durchführung des erfindungsgemäßen Verfahrens weitere Informationen, wie z.B. Patientenbilder, Bemaßungsdaten, Funktionsdaten und/oder sonstige Referenzdaten, Soll-Werte, Standard-Werte und/oder allgemeine patientenunspezifische Daten und/oder Informationen zu Implantaten, wie beispielsweise 2D- und 3D-Modelle von Implantaten, Herstellerinformationen, Artikelnummer, Bemaßung, Material, Verwendungsanweisungen etc., der Implantate, und/oder Skizzen sowie Norm-, Ideal- oder Sollwerte der menschlichen oder tierische Anatomie zugänglich gemacht werden.

Patientenspezifische und allgemeine Informationen können insbesondere mit den mehreren mehrdimensionalen Patientenbildern dargestellt, abgebildet und/oder abgespeichert werden.

Die Darstellungen und die dazugehörigen Daten umfassen vorzugsweise mehrdimensionale Bilder.

Die mehrdimensionalen Bilder sind vorzugsweise 2D-Darstellungen oder 3D-Darstellungen.

## Patentansprüche

1. Verfahren, bei dem zur Durchführung einer Operation an einem menschlichen oder einem tierischen Körper,
- einem Visualisierungsmittel zur Kombinierung von natürlichen Wahrnehmungen wenigstens eines Bereiches des Körpers eines Benutzers mit wenigstens einer künstlich erzeugten Darstellung,
- zur Erzeugung der künstlichen Darstellung Daten bereitgestellt werden, wobei
- die künstliche Darstellung eine Referenzdarstellung zu dem Bereich des Körpers ist und
- die künstliche Darstellung zur Durchführung der Operation mit Hilfe des Visualisierungsmittels optisch auf die natürliche Wahrnehmung des Bereichs des Körpers projiziert wird.

2. Verfahren nach Anspruch 1 **dadurch gekennzeichnet, dass** das Visualisierungsmittel zur Kombinierung der natürlichen Wahrnehmung des Bereichs des Körpers durch den Benutzer mit wenigstens einer künstlich erzeugten Darstellung eine augmented-Reality-Brille ist.

3. Verfahren nach Anspruch 1 **dadurch gekennzeichnet, dass** das Visualisierungsmittel zur Kombinierung der natürlichen Wahrnehmung des Bereichs des Körpers durch den Benutzer mit wenigstens einer künstlich erzeugten Darstellung eine Rechnereinheit umfasst.

4. Verfahren nach Anspruch 1 **dadurch gekennzeichnet, dass** das Visualisierungsmittel zur Kombinierung der natürlichen Wahrnehmung des Bereichs des Körpers durch den Benutzer mit wenigstens einer künstlich erzeugten Darstellung eine Kamera umfasst.

5. Verfahren nach Anspruch 1 **dadurch gekennzeichnet, dass** die Daten zur Erzeugung der künstlichen Darstellung von einer Bildassistenzeinrichtung bereitgestellt werden.

6. Verfahren nach Anspruch 1 **dadurch gekennzeichnet, dass** die künstlich erzeugte Darstellung eine Skala ist.

7. Verfahren nach Anspruch 1 **dadurch gekennzeichnet, dass** die künstlich erzeugte Darstellung eine Schnittlinie an und/oder eine Bohrlinie in einem Bereich des Körpers vorgibt.

8. Verfahren nach Anspruch 1 **dadurch gekennzeichnet, dass** die künstlich erzeugte Darstellung die wenigstens zweidimensionale Abbildung wenigstens eines Implantats zeigt.

9. Verfahren nach Anspruch 1 **dadurch gekennzeichnet, dass** die künstlich erzeugte Darstellung den Eintrittswinkel und/oder die Eindringtiefe des Implantats gegenüber einem Bereich des Körpers vorgibt.

10. Verfahren nach Anspruch 1 **dadurch gekennzeichnet, dass** mit Hilfe der künstlich erzeugten Darstellung des Implantats eine Auswahl eines aus wenigstens zwei Implantaten durchgeführt wird, wobei sich die Implantate in Art, Umfang und/oder Material unterscheiden.

11. Verfahren nach Anspruch 1 **dadurch gekennzeichnet, dass** die Referenzdarstellung einen Zustand des Bereichs des Körpers wiedergibt, der dem Zustand des Bereichs in einer pre-operativen Phase entspricht.

12. Verfahren nach Anspruch 1 **dadurch gekennzeichnet, dass** die künstliche Darstellung mit Hilfe wenigstens eines Referenzmarkers deckungsgleich auf die natürliche Wahrnehmung des Bereichs des Körpers projiziert wird.

13. Verfahren nach Anspruch 1 **dadurch gekennzeichnet, dass** bei einer Abweichung einer optischen Projektion der künstlichen Darstellung von der natürlichen Wahrnehmung des Bereichs des Körpers durch den Benutzer das Visualisierungsmittel eine optische oder akustische Warnmeldung auslöst.

14. Verwendung des Visualisierungsmittels nach einem oder mehreren der vorausgehenden Ansprüche, zur Durchführung einer Operation an einem menschlichen oder einem tierischen Körper.

15. System zur Ausführung des Verfahrens, nach einem der vorausgehenden Ansprüche.
